# EUROPÄISCHE PATENTSCHRIFT

(11) **EP 1 297 811 B1**
(45) Veröffentlichungstag und Bekanntmachung des Hinweises auf die Patenterteilung: **24.08.2005**
(21) Anmeldenummer: 02020788.2
(22) Anmeldetag: 17.09.2002
(51) Int. Cl.: H04N 7/18, A61B 5/11

(54) **Überwachungssystem zur Verlaufskontrolle neurologischer Erkrankungen**
Surveillance system for the monitoring of neurologic diseases
Système de surveillance de maladies neurologiques

(30) Priorität: 21.09.2001 DE 10146680
(43) Veröffentlichungstag der Anmeldung: 02.04.2003
(73) Patentinhaber: Rzesnitzek, Alexander, 56068 Koblenz (DE); Schmitt, Eberhard, Dr., 56068 Koblenz (DE)
(72) Erfinder: Rzesnitzek, Alexander, 56068 Koblenz (DE); Schmitt, Eberhard, Dr., 56068 Koblenz (DE)
(74) Vertreter: Bernhardt, Winfrid

(56) Entgegenhaltungen:
- WO-A-01/88836
- US-A- 4 631 676
- US-A- 4 802 005
- US-A- 5 441 047
- US-A- 5 980 429

## Beschreibung

Die Erfindung betrifft ein Überwachungssystem zur Verlaufskontrolle neurologischer Erkrankungen, insbesondere zur Überwachung von Patienten mit gestörter Bewegungsmotorik, gemäß dem Oberbegriff des Anspruchs 1.

Im Rahmen der Verlaufskontrolle neurologischer Erkrankungen stellt sich unter anderem die Aufgabe, die Wirkung von Medikamentengaben zu beobachten. Zum Beispiel ist bei einem Teil der Parkinson-Patienten nach etwa vier Jahren eine Verkürzung der Wirkdauer zu beobachten, die sich anfangs noch durch Verringerung der Zeitabstände zwischen den Medikamentengaben ausgleichen lässt. Im weiteren Krankheitsverlauf ändert sich die Wirkdauer unregelmäßig, wobei nach einer Medikamenteneinnahme sogar Überbewegungen auftreten können, die in Einzelfällen so heftig sind, dass sie eine Indikation für einen epilepsiechirurgischen Eingriff darstellen.

So sind Fluktuationen von Medikamentenwirkungen ein häufiger Grund, Patienten mit gestörter Bewegungsmotorik stationär aufzunehmen, um sie nach der Medikamentenaufnahme beobachten zu können. Wünschenswert sind auf Tage etwa in Zeitabständen von Stunden verteilte Beobachtungstermine. Im Alltag lassen sich diese selbst bei stationärer Behandlung jedoch kaum in ausreichendem Umfang organisieren. Beurteilungen basieren auf wenigen Stichproben, die noch dazu von verschiedenen Personen nach nicht ausreichend standardisierten Kriterien erhoben werden.

Ein Überwachungssystem, das die Merkmale des Oberbegriffs des Anspruchs 1 aufweist, geht aus der US 5,441, 047 hervor. Bei diesem System können Bilder von Patienten in einer Beobachtungsstation zu einer Auswertzentrale übertragen werden, und die übertragenen Bilder aufgezeichnet und zu einem gewünschten Zeitpunkt auf einem Bildschirm dargestellt werden.

Der vorliegenden Erfindung liegt die Aufgabe zugrunde, die Behandlungsmöglichkeiten neurologischer Erkrankungen, insbesondere von Patienten mit gestörter Bewegungsmotorik, zu verbessern.

Diese Aufgabe wird durch ein Überwachungssystem mit den Merkmalen des Anspruchs 1 gelöst.

In diesem Überwachungssystem begibt sich der Patient zu vorbestimmten Zeitpunkten nach der Medikamenteneinnahme, z.B. in Abständen von etwa 1 Stunde, in die Beobachtungsstation, wo er in dem durch die Kamera zu erfassenden Objektfeld, jeweils eine bestimmte, vorher mit ihm vereinbarte Bewegung ausführt und sich z.B. entlang eines am Boden markierten Laufstegs bewegt. Die ggf. über einen Tag gespeicherte Bildinformation wird durch die Bildverarbeitungseinrichtung verdichtet. Der behandelnde Arzt kann die in Zeitabständen aufgenommenen bewegten Bilder an der Beobachtungsstation unmittelbar aufeinanderfolgend in der Zusammenschau betrachten. Im Beobachtungszeitraum braucht er selbst keine Beobachtungstermine wahrzunehmen. Die Betrachtung der Bildsequenz in der Zusammenschau erlaubt es, Änderungen der Bewegung des Patienten genauer zu erfassen, als dies durch direkte Beobachtung des Patienten in Zeitabständen möglich wäre. Das System letztlich eine genaue Quantifizierung und Objektivierung von Wirkfluktuationen. Bei verringertem Personalaufwand lässt sich die Qualität der Verlaufskontrolle neurologischer Erkrankungen deutlich erhöhen.

Die Speicherung und Verarbeitung kann in Zuordnung zu Identifizierungsdaten und letztlich Patientendateien vorgenommen werden.

In einer bevorzugten Ausführungsform der Erfindung sind mehrere Beobachtungsstationen und eine die Bildausgabeeinrichtung umfassende Auswertzentrale vorgesehen, wobei die Auswertzentrale die Bildausgabeeinrichtung enthält und vorzugsweise durch einen mit den Beobachtungsstationen vernetzten Computer gebildet ist.

Die einzelnen Beobachtungsstationen können ihrerseits mit einer Computereinheit ausgestattet sein, die mit der elektronischen Kamera verbunden ist, deren Bilddaten sie empfängt und vorverarbeitet. Entsprechend kann diese Computereinheit die Speichereinrichtung und die Verarbeitungseinrichtung bilden und zum Datenverkehr mit der Auswertzentrale vorgesehen sein.

In weiterer Ausgestaltung der Erfindung kann die Bildaufnahme durch die elektronische Kamera über die Patientenidentifizierungseinrichtung steuerbar sein. Beispielsweise kann die Patientenidentifizierungseinrichtung ein Lesegerät für einen vom Patienten getragenen Transponder aufweisen. Mit dem Auslesen des Transponders wird nach einer bestimmten Wartezeit, innerhalb der sich der Patient zum Objektfeld begeben kann, die Kamera in Gang gesetzt wird, was dem Patienten z.B. durch ein Lichtsignal anzeigbar ist, auf das er dann die vereinbarten Bewegungen ausführt.

Vorteilhaft ist ferner eine programmierbare, vom Patienten mitgeführte Signalgebereinrichtung vorgesehen, die den Patienten zu vorgewählten Zeiten zur Einnahme von Medikamenten und zum Aufsuchen der Beobachtungsstation auffordern. Insbesondere ist diese Signalgebereinrichtung anhand in die Computereinheit oder den Personalcomputer eingegebener Daten automatisch über entsprechende Zusatzgeräte programmierbar.

In weiterer vorteilhafter Ausgestaltungen der Erfindung können Einrichtungen zur automatischen Koordinierung von Beobachtungsterminen vorgesehen sein, welche Doppelbelegungen der Beobachtungsstationen verhindern und den, bezogen auf eine Medikamentengabe jeweils optimalen Ausweichtermin für einen Patienten aussuchen.

Zweckmäßig ist an der Beobachtungsstation eine Einrichtung zur Ausgabe von Informationen für den Patienten vorgesehen, z.B. ein Drucker, welcher eine im Ergebnis der Untersuchungen geänderte Medikamentierung ausdruckt.

In weiterer Ausgestaltung der Erfindung kann die Bildverarbeitungseinrichtung über eine Zusammenstellung der in Zeitabständen aufgenommenen Bilder zu der Bildsequenz hinaus zur Bearbeitung der aufgenommenen Bilder selbst ausgebildet sein. Zum Beispiel können die einzelnen, zu verschiedenen Zeiten aufgenommenen bewegten Bilder jeweils in die Sequenz noch weiter verkürzende Zeitrafferaufnahmen überführt werden, oder es werden zwischen den einzelnen Bildern automatische Vergleiche durchgeführt und z.B. Bildüberlagerungen dargestellt, welche Änderungen der aufgezeichneten Bewegungen deutlich hervortreten lassen.

Die Erfindung soll nun anhand eines Ausführungsbeispiels und der beiliegenden, sich auf dieses Ausführungsbeispiel beziehenden Zeichnungen näher erläutert werden. Es zeigen:

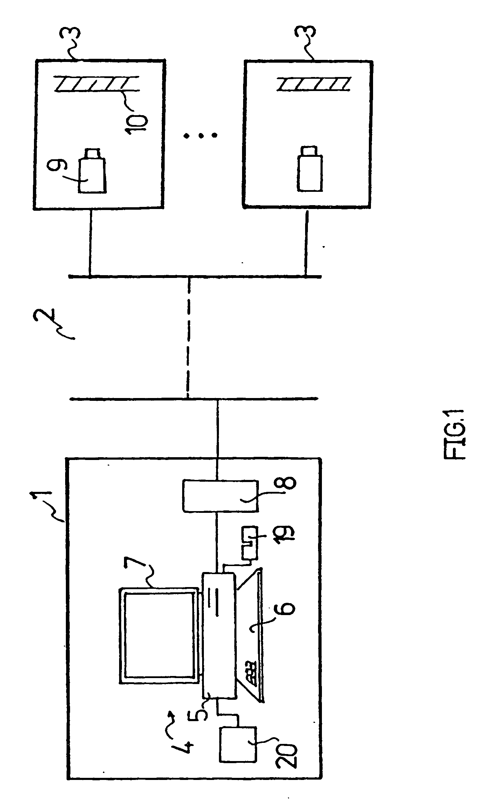

- Fig. 1: eine Schemadarstellung eines Überwachungssystems nach der Erfindung, und
- Fig. 2: eine in dem Überwachungssystem von Fig. 1 verwendete Beobachtungsstation.

Eine Auswertzentrale 1 ist über ein Netz 2, z.B. ein das Telefonnetz einschließendes Netz, oder direkt mit einer Vielzahl von Beobachtungsstationen 3 verbunden.

Die Auswertzentrale 1 umfasst einen Personalcomputer 4, der in üblicher Weise eine zentrale Computereinheit 5, einen Monitor 6 und eine Eingabetastatur 7 aufweist.

Über ein Modem 8 kann der Personalcomputer 4 über das Netz 2 in Datenverkehr mit den Beobachtungsstationen 3 treten.

Die Beobachtungsstationen 3 sind jeweils mit einer elektronischen Kamera 9 ausgerüstet. Eine streifenartige Bodenmarkierung 10 kennzeichnet ein Objektfeld, auf das die Kamera 9 ausgerichtet ist. Die Kamera 9 ist mit einem Weitwinkelobjektiv versehen.

An den Personalcomputer angeschlossen sind ferner ein Kartenleser 19 und eine Programmiereinheit 20.

Standort der Auswertzentrale 1 kann z.B. eine neurologische Klinik sein. Die Beobachtungsstationen 3 sind z.B. auf Altenheime oder andere patientennahe Einrichtungen verteilt.

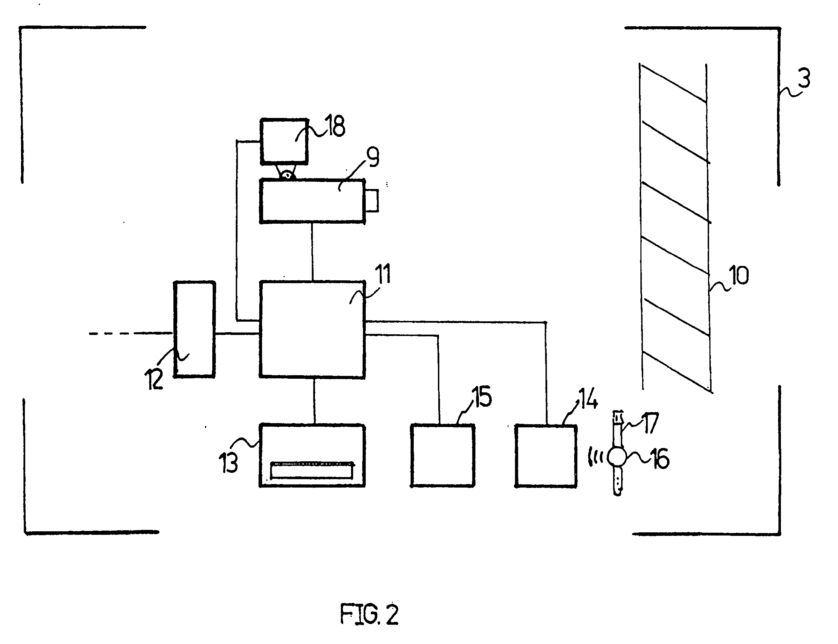

Es wird nun auf Fig. 2 Bezug genommen, wo eine Beobachtungsstation 3 detaillierter dargestellt ist.

Die zur Aufnahme bewegter Bilder geeignete, mit einem Weitwinkelobjektiv versehene elektronische Kamera 9 ist mit einer zentralen Computereinheit 11 verbunden, die wie der Computer 4 in der Auswertzentrale 1 durch einen Personalcomputer gebildet sein kann.

Die Computereinheit 11 bildet eine zentrale Steuerung für die Beobachtungsstation 3. Über ein Modem 12 kann sie in Datenverkehr mit dem Personalcomputer 4 in der Auswertstation 1 treten.

An die Computereinheit 11 ist ferner ein Drucker 13 angeschlossen.

Mit der Computereinheit 11 stehen darüber hinaus ein Transponderleser 14 und eine Programmiereinheit 15 in Verbindung. Der Transponderleser 14 dient zum Auslesen eines Transponders, der in einer am Arm eines Patienten zu tragenden Baueinheit 16 enthalten ist. Die an einem Armband ,17 befestigte Baueinheit 16 enthält ferner einen Signalgeber, dessen Signale den Patienten dazu auffordern, zu vorbestimmten Zeiten Medikamente einzunehmen und die Beobachtungsstation 3 aufzusuchen.

Dieser Signalgeber ist über die mit der Computereinheit 11 verbundene Programmiereinheit 15 automatisch programmierbar.

Das Bezugszeichen 18 weist schließlich auf eine mechanische Bewegungseinrichtung für die Kamera 9 hin, welche über die Computereinheit 11 steuerbar ist.

Ein zu behandelnder Patient meldet sich in einer Klinik bei der Auswertstation 1 zur Behandlung an, wobei z.B. dessen Versicherungskarte durch den Kartenleser 19 eingelesen und eine Patientendatei angelegt oder geöffnet wird. Der Patient erhält ein Armband 17 mit der den Transponder und die Signalgebereinrichtung enthaltenden Baueinheit 16. Der Transponder liefert einen Identifikationscode. Doppelvergaben dieses Codes sind ausgeschlossen. Der Code wird der Patientendatei zugeordnet.

Der behandelnde Arzt gibt über die Tastatur 6 ferner Einnahmezeitpunkte für Medikamente sowie Zeiten, zu denen der Patient eine für ihn zuständige Behandlungsstation 3 aufsuchen soll. Anhand dieser Daten wird über die Programmiereinheit 20 der Signalgeber in der Baueinheit 16 automatisch programmiert. In dem betreffenden Ausführungsbeispiel wird zur Programmierung eine Steckverbindung zwischen der Programmiereinheit 20 und der Baueinheit 16 hergestellt. Eine solche Programmierung könnte jedoch auch durch drahtlosen Datenaustausch erfolgen.

Durch den Signalgeber erhält der Patient Signale, die ihn auffordern, ein Medikament einzunehmen und darauffolgend in Zeitabständen weitere Signale, die ihn zum Aufsuchen einer der Behandlungsstationen 3 auffordern.

Beim Aufsuchen der Behandlungsstation 3 meldet sich der Patient an, indem er den Signalgeber durch den Transponderleser 14 auslesen lässt. Für den so identifizierten Patienten wird in der Computereinheit 11 dessen Patientendatei geöffnet, welche in dem betreffenden Ausführungsbeispiel von der Auswertzentrale 1 auf die betreffende Computereinheit 6 über das Netz 2 übertragen wurde.

Gleichzeitig wird durch die Identifizierung die Kamera 9 aktiviert und nach einer Wartezeit der Betrieb der Kamera durch eine Signalleuchte an der Kamera angezeigt. Zweckmäßig ist der Transponderteser 14 nahe bei der Bodenmarkierung 10 angeordnet, so dass sich der Patient bereits in der Ausgangsposition für eine vorher vereinbarte, in jedem Beobachtungszyklus auszuführende Bewegung befindet und auf das Signal an der Kamera sofort mit der Bewegung beginnen kann. In dem betreffenden Ausführungsbeispiel läuft ein Parkinson-Patient jeweils entlang der Bodenmarkierung 10. Natürlich sind beliebige andere, durch die Kamera 9 aufzunehmende Bewegungen denkbar. Gegebenenfalls wird die Kamera durch die Bewegungseinrichtung 18 nachgeführt, wobei es denkbar ist, eine solche Nachführung über eine Erfassung der Bewegung (Motion Detektion) bzw. das Transpondersignal zu steuern, indem z.B. entlang der Bodenmarkierung 11 den Transponder ansprechende Sender-/Empfänger-einrichtungen angeordnet werden.

Die Computereinheit 11 speichert patientenbezogen digital die ihr von der Kamera 9 zugeführten Bildsignale in Zuordnung zur Uhrzeit und legt die Signale von Beobachtung zu Beobachtung so im Speicher ab, dass bei einer späteren Auslesung durch die Auswertzentrale 1 auf dem Monitor 7 eine Sequenz der in Zeitabständen von z.B. 1 Stunde jeweils über z.B. 5 Sekunden aufgenommenen bewegten Bilder in zusammenhängender Form darstellbar ist.

Der behandelnde Arzt wertet diese Bilder in der Auswertzentrale 1 z.B. tageweise aus, wobei er durch die Verdichtung der in Zeitabständen über Tage gesammelten Bilder auf Bildsequenzen von kurzer Dauer in der Lage ist, sich gleichzeitig um eine Vielzahl von Patienten zu kümmern.

Der Personalcomputer 4 der Auswertzentrale 1 kann zu einer weitergehenden Bildbearbeitung programmiert sein, indem die einzelnen Bilder einer Sequenz z.B. im Zeitrafferverfahren dargestellt werden, die Reihenfolge von Bildern der Sequenz vertauscht wird oder mehrere Bilder der Sequenz in Überlagerung gezeigt werden.

Nach einer gewissen Behandlungszeit können neue Medikationen einschließlich der Einnahmezeitpunkte und Beobachtungszeiten festgelegt werden, die der behandelnde Arzt über die Tastatur 6 eingibt. Über das Netz 2 zur Computereinheit 11 übertragene Informationen können mit Hilfe des Druckers 13 für den Patienten ausgegeben werden. Ferner besteht für den Patienten die Möglichkeit, an der Beobachtungsstation mit Hilfe der Programmiereinheit 15 automatisch eine Neuprogrammierung seiner Signalgebereinrichtung durchführen.

Es versteht sich, dass die beschriebenen Gerätefunktionen weitgehend durch die Programmierung des Personalcomputers 4 und der Computereinheit 11 gestützt sind, wobei die Software bei gleicher Funktion in unterschiedlicher Weise verteilt auf die beiden miteinander kommunizierenden Computer verteilt sein kann. Es wäre z.B. denkbar, die Bildspeicherung und -verarbeitung nur in der Auswertzentrale 1 durchzuführen. Um dort den Datenanfall in Grenzen zu halten, ist es jedoch zweckmäßiger, in den Beobachtungsstationen 3 eine möglichst weitgehende Datenvorverarbeitung durchzuführen.

## Patentansprüche

1. Überwachungssystem zur Verlaufskontrolle neurologischer Erkrankungen, insbesondere zur Überwachung von Patienten mit gestörter Bewegungsmotorik, wobei das System wenigstens eine Beobachtungsstation (3) mit einer elektronischen Kamera (9) für die Aufnahme bewegter Bilder sowie Einrichtungen (10) zur Markierung eines durch die Kamera (9) zu erfassenden Objektfeldes,
eine Speichereinrichtung zur digitalen Speicherung durch die Kamera (9) in der Beobachtungsstation (3) in vorgewählten Zeitabständen von einem Patienten aufzunehmender Bilder und
eine Bildausgobeeinrichtung (7) für die Darstellung der Bilder aufweist,
**dadurch gekennzeichnet,**
**dass** eine Bildverarbeitungseinrichtung zur Verarbeitung der in Zeitabständen aufgenommenen Bilder zu einer gegenüber dem Beobachtungszeitraum verkürzten Bildsequenz vorgesehen ist,
**dass** die Bildausgabeeinrichtung (7) zur Darstellung der Bildsequenz vorgesehen ist, und
**dass** das System zur Überwachung einer Vielzahl von zeitversetzt die wenigstens eine Beobachtungsstation (3) aufsuchenden Patienten vorgesehen ist, wobei die Beobachtungsstation (3) Einrichtungen (14) zur automatischen Patientenidentifixierung aufweist und die Speichereinrichtung und Bildverarbeitungseinrichtung zur patientenbezogenen Bildspeicherung bzw. -verarbeitung in Zuordnung zu Identifizierungsdaten vorgesehen sind.

2. System nach Anspruch 1,
**gekennzeichnet durch** mehrere Beobachtungsstationen (3) und in räumlicher Trennung zu den Beobachtungsstationen eine mit diesen zwecks Datenübertragung verbindbare, die Bildausgabeeinrichtung (7) umfassende Auswertzentrale.

3. System nach Anspruch 2,
**dadurch gekennzeichnet,**
**dass** die Auswertzentrale durch einen Computer, vorzugsweise Personalcomputer (4), gebildet ist.

4. System nach einem der Ansprüche 1 bis 3,
**dadurch gekennzeichnet,**
**dass** die Beobachtungsstation eine mit der elektronischen Kamera verbundene Computereinheit (11) umfasst.

5. System nach Anspruch 4,
**dadurch gekennzeichnet,**
**dass** die Computereinheit (11) zum Datenverkehr mit der Auswertzentrale (1) vorgesehen ist und die Speichereinrichtung und die Verarbeitungseinrichtung umfasst.

6. System nach einem der Ansprüche 1 bis 5,
**dadurch gekennzeichnet,**
**dass** die elektronische Kamera (9) durch Engaben in die Patientenidentifizierungseinrichtung (14) steuerbar ist.

7. System nach einem der Ansprüche 1 bis 6,
**gekennzeichnet durch** einen programmierbaren, vom Patienten mitgeführten Signalgeber zur Abgabe von den Patienten zur Medikamenteneinnahme und/oder zum Aufsuchen der Beobachtungsstation auffordernden Signalen.

8. System nach Anspruch 7,
**dadurch gekennzeichnet,**
**dass** Einrichtungen (15,20) zur automatischen Programmierung der Signalgebereinrichtung anhand eingegebener Daten vorgesehen sind.

9. System nach einem der Ansprüche 1 bis 8,
**dadurch gekennzeichnet,**
**dass** Einrichtungen zur automatischen Koordinierung von Beobachtungsterminen vorgesehen sind.

10. System nach einem der Ansprüche 1 bis 9,
**dadurch gekennzeichnet,**
**dass** die Bildverarbeitungseinrichtung über eine Zusammenstellung der in Zeitabständen aufgenommenen Bilder zu der Bildsequenz hinaus zur Bearbeitung aufgenommener Bilder selbst vorgesehen ist.

## Claims

1. Monitoring system for monitoring the progress of neurological illnesses, in particular for monitoring patients with a disturbed movement motor system, with the monitoring system having at least one observation station (3) with an electronic camera (9) for recording moving images, as well as devices (10) for marking an object field to be recorded by the camera (9),
having a storage device for digital storage of images of a patient to be recorded by the camera (9) in the observation station (3) at predetermined time intervals, and
having an image output device (7) for displaying the images,
**characterized**
**in that** an image processing device is intended to process the images which are recorded at time intervals to form an image sequence which is shorter than the observation time period,
**in that** the image output device (7) is intended to display the image sequence, and
**in that** the system is intended to monitor a large number of patients who visit the at least one observation station (3) at different times, with the observation station (3) having devices (14) for automatic patient identification, and the storage device and image processing device being intended for patient-related image storage and processing associated with identification data.

2. System according to Claim 1,
**characterized by** two or more observation stations (3) and an evaluation control centre, which can be connected to these observation stations for data transmission and has the image output device (7), physically separated from the observation stations.

3. System according to Claim 2,
**characterized**
**in that** the evaluation control centre is formed by a computer, preferably by a personal computer (4).

4. System according to one of Claims 1 to 3,
**characterized**
**in that** the observation station has a computer unit (11) which is connected to the electronic camera.

5. System according to Claim 4,
**characterized**
**in that** the computer unit (11) is intended to interchange data with the evaluation control centre (1) and comprises the storage device and the processing device.

6. System according to one of Claims 1 to 5,
**characterized**
**in that** the electronic camera (9) can be controlled by inputs into the patient identification device (14).

7. System according to one of Claims 1 to 6,
**characterized by** a programmable signal transmitter, which is carried by the patient, for emission of signals which request the patients to take medicaments and/or to visit the observation station.

8. System according to Claim 7,
**characterized**
**in that** devices (15, 20) are provided for automatic programming of the signal transmitter device on the basis of entered data.

9. System according to one of Claims 1 to 8,
**characterized**
**in that** devices are provided for automatic coordination of observation appointments.

10. System according to one of Claims 1 to 9,
**characterized**
**in that** the image processing device is intended, in addition to formation of the image sequence of the images recorded at time intervals, to process the recorded images themselves.

## Revendications

1. Système de surveillance pour le contrôle de l'évolution de maladies neurologiques, en particulier pour la surveillance de patients présentant un trouble de la motricité, le système présentant au moins un poste d'observation (3) avec une caméra électronique (9) pour l'enregistrement d'images en mouvement ainsi que des dispositifs (10) pour marquer un champ d'objet à saisir par la caméra (9), un dispositif d'enregistrement pour l'enregistrement digital d'images à prendre d'un patient par la caméra (9) dans le poste d'observation (3) à des intervalles de temps prédéfinis et un dispositif de sortie (7) des images pour la représentation des images, **caractérisé en ce qu'**on a prévu un dispositif de traitement d'images pour traiter les images prises à certains intervalles en une séquence d'images plus courte par rapport au laps de temps d'observation, **en ce que** le dispositif de sortie (7) des images est prévu pour la représentation de la séquence d'images et **en ce que** le système pour la surveillance est prévu pour une multitude de patients qui se présentent devant ledit au moins un poste d'observation (3) de manière décalée dans le temps, le poste d'observation (3) présentant des dispositifs (14) pour l'identification automatique des patients et le dispositif d'enregistrement et le dispositif de traitement d'images étant prévu pour l'enregistrement et le traitement d'images concernant les patients en les attribuant aux données d'identification.

2. Système selon la revendication 1, **caractérisé par** plusieurs postes d'observation (3) et une centrale d'analyse séparée dans l'espace par rapport aux postes d'observation, pouvant être raccordée à ceux-ci en vue du transfert des données, comprenant le dispositif de sortie (7) des images.

3. Système selon la revendication 2, **caractérisé en ce que** la centrale d'analyse est formée par un ordinateur, de préférence un ordinateur personnel (4).

4. Système selon l'une quelconque des revendications 1 à 3, **caractérisé en ce que** le poste d'observation comprend une unité informatique (11) raccordée à la caméra électronique.

5. Système selon la revendication 4, **caractérisé en ce que** l'unité informatique (11) est prévue pour le transport de données avec la centrale d'analyse (1) et comprend le dispositif d'enregistrement et le dispositif de traitement.

6. Système selon l'une quelconque des revendications 1 à 5, **caractérisé en ce que** la caméra électronique (9) peut être contrôlée par des données introduites dans le dispositif d'identification des patients (14).

7. Système selon l'une quelconque des revendications 1 à 6, **caractérisé par** un émetteur de signaux programmable, emporté par les patients pour émettre des signaux incitant les patients à prendre un médicament et/ou à se présenter au poste d'observation.

8. Système selon la revendication 7, **caractérisé en ce qu'**on a prévu des dispositifs (15, 20) pour la programmation automatique du dispositif d'émission de signaux à l'aide des données introduites.

9. Système selon l'une quelconque des revendications 1 à 8, **caractérisé en ce qu'**on a prévu des dispositifs pour la coordination automatique des moments d'observation.

10. Système selon l'une quelconque des revendications 1 à 9, **caractérisé en ce que** le dispositif de traitement d'images est prévu pour le rassemblement des images prises à des intervalles dans le temps en une séquence d'images et en outre pour le traitement des images enregistrées elles-mêmes.
